# FASCICULE DE BREVET EUROPEEN

(11) **EP 2 535 080 B1**
(45) Date de publication et mention de la délivrance du brevet: **03.12.2014**
(21) Numéro de dépôt: 12171593.2
(22) Date de dépôt: 12.06.2012
(51) Int. Cl.: A61N 1/04, A61N 1/32, A61N 1/36

(54) **Electrode combinée pour le traitement de l'hyperhidrose axillaire**
Kombinierte Elektrode für die Behandlung von Hyperhidrosis axillae
Combined electrode for the treatment of axillary hyperhidrosis

(30) Priorité: 14.06.2011 FR 1155151
(43) Date de publication de la demande: 19.12.2012
(73) Titulaire: I2M, 14000 Caen (FR)
(72) Inventeur: Desnos, Philippe, 14000 Caen (FR); Hamel, Patrick, 14000 Caen (FR)
(74) Mandataire: Maillet, Alain

(56) Documents cités:
- WO-A1-94/08656
- WO-A1-99/39635
- DE-A1-102007 011 363
- US-A- 1 480 353
- US-A1- 2007 213 788
- US-A1- 2008 255 480

## Description

La présente invention concerne une électrode combinée pour le traitement de l'hyperhidrose, en particulier pour l'hyperhidrose axillaire, ainsi qu'un dispositif pour le traitement de l'hyperhidrose axillaire comportant une telle électrode combinée.

L'hyperhidrose désigne la production excessive de sueur par un individu.

L'hyperhidrose est soignée depuis de nombreuses années par ionophorèse.

Le dispositif de l'état de la technique qui permet de soigner l'hyperhidrose axillaire par ionophorèse comprend:
- un générateur électrique fonctionnant sur piles ou batteries,
- pour chaque aisselle, une électrode combinée comportant elle-même une électrode électriquement conductrice reliée électriquement au générateur électrique, et un manchon en éponge recouvrant ladite électrode.

L'électrode prend la forme d'une plaque métallique souple qui doit être pliée pour épouser la forme de l'aisselle.

La personne devant être soignée, plie ainsi chaque plaque métallique et positionne une dans chacun des creux axillaires, et ainsi un circuit électrique fermé est réalisé. Le courant électrique délivré par le générateur électrique a alors un trajet préférentiel en direction des glandes sudoripares.

Bien qu'un tel dispositif donne de bons résultats, il présente de nombreux désavantages.

En premier lieu, le pliage ne permet pas d'épouser parfaitement la forme du creux axillaire, et il arrive même que certaines personnes utilisent un tel dispositif sans plier les plaques métalliques.

Dans un cas comme dans l'autre, l'efficacité du traitement n'est pas optimale.

Le document US-A-1,480,353 divulgue une électrode comportant une boule constituée de tissus et enveloppée dans un filet en métal. La boule intérieure n'est pas une électrode métallique et n'est pas électriquement conductrice.

Un objet de la présente invention est de proposer une électrode combinée pour le traitement de l'hyperhidrose axillaire qui ne présente pas les inconvénients de l'art antérieur et qui en particulier permet d'améliorer l'efficacité du traitement.

A cet effet, est proposée une électrode combinée pour le traitement de l'hyperhidrose axillaire, telle que définie dans la revendication 1.

Il est également proposé un dispositif pour le traitement de l'hyperhidrose axillaire comportant un générateur électrique et deux électrodes combinées selon l'une des variantes précédentes, chaque électrode métallique étant reliée électriquement audit générateur électrique.

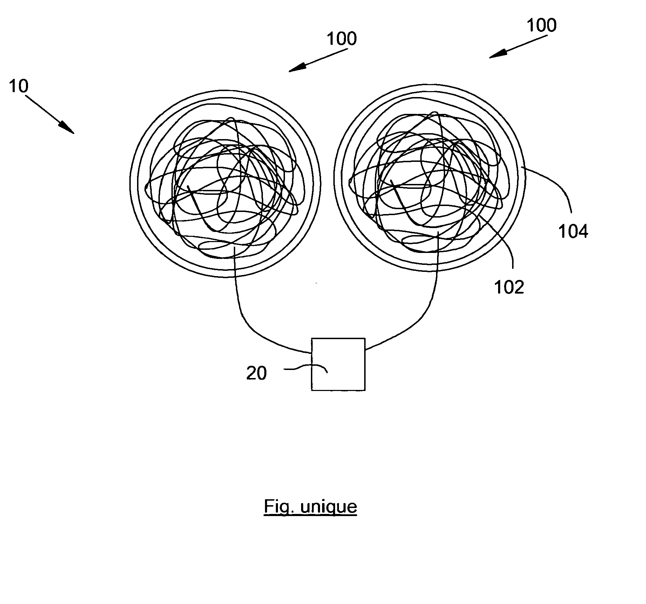

Les caractéristiques de l'invention apparaîtront plus clairement à la lecture de la description suivante d'un exemple de réalisation, ladite description étant faite en relation avec la figure unique jointe qui montre dispositif pour le traitement de l'hyperhidrose axillaire selon l'invention.

La figure unique montre un dispositif 10 pour le traitement de l'hyperhidrose axillaire qui comporte un générateur électrique 20 délivrant un courant dont l'intensité peut être modifiée, et deux électrodes combinées 100 qui sont ici vues en coupe. Chaque électrode combinée 100 est reliée électriquement au générateur électrique 20.

Chaque électrode combinée 100 comprend:
- un électrode métallique 102 reliée électriquement au générateur électrique 20, et
- une couche de transfert 104 recouvrant ladite électrode métallique 102 et constituée d'un matériau conducteur.

L'électrode métallique 102 prend la forme d'une boule sensiblement sphérique qui est constituée d'un fil métallique.

Le fil métallique est conformé pour former une boule souple, c'est-à-dire que sa forme peut se modifier sous l'action d'une force.

La souplesse est obtenue par le fait que le fil métallique est fin et que la boule est aérée, c'est-à-dire que le fil métallique ne remplit pas tout le volume de la boule.

La boule se présente par exemple sous la forme d'une boule fabriquée par tricotage d'un fil métallique et ressemble ainsi à une éponge métallique.

Le fil métallique est par exemple en inox ou en aluminium.

La couche de transfert 104 est également souple et prend la forme d'un sac également en forme de sphère.

La couche de transfert 104 peut être réalisée en matériau absorbant l'eau du type éponge. L'épaisseur de la couche de transfert 104 est de l'ordre de 0,5 à 3 mm. Le matériau absorbant est par nature isolant électriquement mais lorsqu'il est imbibé d'eau, il devient conducteur. Selon un autre mode de réalisation ne faisant pas partie de la présente invention, la couche de transfert 104 peut être réalisée d'un matériau isolant chargé de particules électriquement conductrices. L'épaisseur de la couche de transfert 104 est de l'ordre de 0,5 à 3 mm. Le matériau isolant forme une matrice qui est par nature isolant électriquement mais la présence des particules électriquement conductrices le rend conducteur.

Selon un premier exemple, le matériau isolant est une mousse et les particules sont des micro particules de carbone.

Pour éviter l'accumulation de sueur dans la mousse, celle-ci est à cellules fermées, comme par exemple une mousse en polyuréthane.

Selon un deuxième exemple, le matériau isolant est un polymère souple. Le polymère souple est par exemple du silicone et les particules sont des micro particules de carbone.

Selon un un autre exemple les dimensions des micro particules sont de l'ordre de 40 nm et leurs masses représentent environ 75% de la masse du matériau isolant.

Une telle électrode combinée ne nécessite plus l'utilisation d'eau pour assurer la continuité électrique de l'ensemble.

Le courant électrique suit les particules conductrices jusqu'aux pores sudoraux et les fuites de courant sont alors très réduites par rapport à l'état de la technique. Cette électrode combinée permet d'appliquer le courant par le chemin le plus court, c'est-à-dire directement à l'entrée des pores sudoraux remplis de sueur.

L'électrode métallique est ainsi enfermée dans ce sac et la consistance particulière de l'électrode métallique fait que sa mise en place dans le creux axillaire est aisée et du fait de la souplesse de la boule, celle-ci épouse au mieux le fond du creux axillaire et le contact avec la peau est amélioré par rapport à une électrode rigide. L'invention est définie par la revendication 1 qui suit.

L'invention a été plus particulièrement décrite dans le cas où l'électrode est constituée d'un fil métallique, mais elle peut être constituée de plusieurs fils métalliques tricotés et imbriqués les uns dans les autres.

## Revendications

1. Electrode combinée (100) pour le traitement de l'hyperhidrose axillaire, ladite électrode combinée (100) comportant une couche de transfert souple (104) et une électrode métallique, enveloppée par ladite couche de transfert, et constituée d'au moins un fil métallique conformé pour former une boule souple, ladite électrode combinée étant **caractérisée en ce que** ladite couche de transfert (104) est constituée d'un matériau absorbant par nature isolant électriquement et conducteur lorsqu'il est imbibé d'eau.

2. Electrode combinée (100) selon la revendication 1, **caractérisée en ce que** ledit ou lesdits fils métalliques sont tricotés.

3. Dispositif pour le traitement de l'hyperhidrose axillaire (10) comportant un générateur électrique (20) et deux électrodes combinées (100) selon l'une des revendications précédentes, chaque électrode métallique (102) étant reliée électriquement audit générateur électrique (20).

## Patentansprüche

1. Kombinierte Elektrode (100) für die Behandlung von Hyperhidrosis axillae, die kombinierte Elektrode (100) umfasst eine weiche Transferschicht (104) und eine metallische Elektrode, die von der Transferschicht umschlossen ist und aus mindestens einem Metalldraht aufgebaut ist, der zur Bildung einer weichen Kugel angeordnet ist, wobei die kombinierte Elektrode **dadurch gekennzeichnet ist, dass** die Transferschicht (104) aus einem saugfähigen Material besteht, welches von Natur aus elektrisch isolierend ist und leitend wird, wenn es mit Wasser befeuchtet wird.

2. Kombinierte Elektrode (100) nach Anspruch 1, **dadurch gekennzeichnet, dass** der oder die Metalldrähte gestrickt sind.

3. Vorrichtung zur Behandlung der Hyperhidrosis axillae (10) mit einem elektrischen Generator (20) und zwei kombinierten Elektroden (100) nach einem der vorgehenden Ansprüche, wobei jede metallische Elektrode (102) elektrisch mit dem elektrischen Generator (20) verbunden ist.

## Claims

1. Combined electrode (100) for the treatment of axillary hyperhidrosis, said combined electrode (100) comprising a flexible transfer layer (104) and a metal electrode enveloped by said transfer layer, and made up of at least one metallic wire formed into a flexible ball, said combined electrode being **characterised in that** said transfer layer (104) is composed of an absorbent material that is by nature electrically insulating and conductive when it is soaked with water.

2. Combined electrode (100) according to claim 1, **characterised in that** said metallic wire or wires are woven.

3. Device for the treatment of axillary hyperhidrosis (10) comprising an electrical generator (20) and two combined electrodes (100) according to one of the preceding claims, each metal electrode (102) being electrically connected to said electrical generator (20).
